# EUROPÄISCHE PATENTANMELDUNG

(11) **EP 0 770 604 A1**
(43) Veröffentlichungstag der Anmeldung: **02.05.1997**
(21) Anmeldenummer: 96116535.4
(22) Anmeldetag: 16.10.1996
(51) Int. Cl.: C07D 263/34

(54) **Verfahren zur Herstellung von Oxazolderivaten**

(30) Priorität: 25.10.1995 CH 3009/95
(71) Anmelder: F. HOFFMANN-LA ROCHE AG, 4070 Basel (CH)
(72) Erfinder: Pauling, Horst, 4103 Bottmingen (CH); Bonrath, Werner, 79115 Freiburg (DE)
(74) Vertreter: Kellenberger, Marcus, Dr.

(57) **Zusammenfassung**

Ein Verfahren zur Herstellung eines 5-Cyano-4-niederalkyl-oxazols durch Dehydratisierung eines entsprechenden 5-Carbamoyl-4-niederalkyl-oxazols ist dadurch gekennzeichnet, dass man die Dehydratisierung mit Titantetrachlorid in Gegenwart einer Base und gegebenenfalls in Gegenwart eines Lösungsmittels durchführt. Das Produkt dieses Verfahrens, das 5-Cyano-4-niederalkyl-oxazol, insbesondere das 5-Cyano-4-methyl-oxazol, ist ein wertvolles Zwischenprodukt bei der Synthese von Pyridoxin (Vitamin B₆).

## Beschreibung

Die vorliegende Erfindung betrifft ein neues Verfahren zur Herstellung von 5-Cyano-4-niederalkyl-oxazolen. Diese Oxazole bilden eine wichtige Stoffgruppe. So ist beispielsweise 5-Cyano-4-methyl-oxazol ein wertvolles Zwischenprodukt bei der Synthese von Pyridoxin (Vitamin B₆).

Es wurden bereits einige Verfahren zur Herstellung von 5-Cyano-4-methyl-oxazol durch Dehydratisierung von 5-Carbamoyl-4-methyl-oxazol beschrieben. So gelingt diese Dehydratisierung beispielsweise in Gegenwart von Phosphorpentoxid. Der Nachteil dieses Verfahrens ist jedoch die geringe Produktausbeute. Dies ist auf die bei dieser Reaktion sehr leicht auftretende Verkohlung zurückzuführen.

Eine Verbesserung dieses Verfahrens gelingt durch die Umsetzung von 5-Carbamoyl-4-methyl-oxazol mit Phosphorpentoxid in Gegenwart von Chinolin als Lösungsmittel [ USP 3 222 374 ]. Auch dieses Verfahren weist Nachteile auf, die sich aus der Giftigkeit des Chinolins, seinem unangenehmen Geruch sowie seiner thermischen Instabilität ergeben. Zudem ist Chinolin ein relativ teures Lösungsmittel. Ein Problem stellt weiterhin die Regenerierung des Chinolins, die Verwendung von stöchiometrischen Mengen Phosphorpentoxid, die Aufarbeitung der Folgeprodukte des benötigten Phosphorpentoxids sowie deren umweltgerechte Entsorgung dar.

Ein weiteres bekanntes Verfahren zur Herstellung von 5-Cyano-4-methyl-oxazol besteht darin, das 5-Carbamoyl-4-methyl-oxazol mit einem Nieder-alkancarbonsäureanhydrid umzusetzen und das Reaktionsgemisch oder das aus diesem isolierte 4-Methyl-5-(N-niederalkanoyl-carbonyl)-oxazol einer Pyrolyse zu unterwerfen [ EP 0 010 697 ]. Die pyrolytische Endstufe hat jedoch gewisse Nachteile; insbesondere treten Korrosionsprobleme mit den Reaktorwerkstoffen auf, und es bilden sich schwer rezyklisierbare Nebenprodukte.

Ein weiteres Verfahren [ USP 4 026 902 ] besteht darin, dass 5-Carbamoyl-4-methyl-oxazol katalytisch unter Erhitzen in Anwesenheit von Phosphorpentoxid auf einem festen Träger zu 5-Cyano-4-methyl-oxazol dehydratisiert wird. Nachteilig bei diesem Verfahren sind die Handhabung von 5-Carbamoyl-4-methyl-oxazol, insbesondere die im Vordergrund stehende Sublimation und damit die Feststoffdosierung des schwerflüchtigen Ausgangsmaterials.

In der US-Patentschrift 4 772 718 wird weiterhin die einstufige Ueberführung von 5-Carbonsäureethylester-4-methyl-oxazol in 5-Cyano-4-methyl-oxazol beschrieben. Bei diesem Verfahren wird der entsprechende Oxazolester in Gegenwart von Ammoniak und einem Zirkonoxid- oder Hafniumoxidkatalysator in der Gasphase zu 5-Cyano-4-methyl-oxazol umgesetzt. Nachteilig ist hier jedoch die Verwendung eines relativ teuren Katalysators sowie - zwecks Erreichen einer optimalen Reaktionsführung- das Einhalten sehr enger Reaktionbedingungen.

Die in der europäischen Patentpublikation 0 492 233 beschriebene Gasphasendehydratisation von 5-Carbamoyl-4-methyl-oxazol zu 5-Cyano-4-methyl-oxazol besitzt den Nachteil einer Reaktionstemperatur von 400°C - 500°C und muss bei einem Druck von 50 bis ca. 300 kPa durchgeführt werden.

Ein Verfahren zur Herstellung von Nitrilen aus Carbonsäureamiden bei niedriger Temperatur wird in Tetrahedron Letters 19, 1501-1502 (1971) beschrieben. Die Dehydratisierung erfolgt durch Umsetzung mit Titantetrachlorid im Ueberschuss. Auf die Dehydratisierung von Oxazolnitril oder strukturnahen Verbindungen wird nicht verwiesen.

Der vorliegenden Erfindung liegt nun die Aufgabe zugrunde, ein Verfahren zur Herstellung von 5-Cyano-4-niederalkyl-oxazolen bereitzustellen, das die Nachteile der vorbekannten Arbeitsweisen nicht aufweist, und gemäss welchem 5-Cyano-4-niederalkyl-oxazol in kurzer Reaktionszeit unter milden Reaktionbedingungen in hoher Ausbeute erhalten wird.

Das erfindungsgemässe Verfahren zur Herstellung eines 5-Cyano-4-niederalkyl-oxazols durch Dehydratisierung eines 5-Carbamoyl-4-niederalkyl-oxazols ist dadurch gekennzeichnet, dass die Dehydratisierung des 5-Carbamoyl-4-niederalkyl-oxazols mit Titantetrachlorid in Gegenwart einer Base und gegebenenfalls in Gegenwart eines Lösungsmittels durchgeführt wird.

Der Ausdruck "niederes Alkyl" bedeutet im Rahmen der vorliegenden Erfindung geradkettige oder verzweigte Alkylreste mit 1 bis 6 Kohlenstoffatomen, wie z.B. Methyl, Ethyl, Propyl, Isopropyl, Butyl, Isobutyl, tert.Butyl, Pentyl, Hexyl und dergleichen. Geradkettige Alkylreste, wie insbesondere Methyl und Ethyl, sind bevorzugt.

Geeignete Lösungsmittel sind niedere aliphatische oder cyclische Ether, z.B. Diethylether bzw. Tetrahydrofuran oder Dioxan, oder niedere aliphatische Nitrile, z.B. Acetonitril. Bevorzugt sind Tetrahydrofuran und Acetonitril.

Als Basen werden, im Rahmen der vorliegenden Erfindung zweckmässigerweise Triethylamin, 4-Methylmorpholin, Pyridin und dergleichen verwendet. Bevorzugt ist Triethylamin.

Die Dehydratisierung kann zweckmässigerweise in einem Temperaturbereich von etwa 0°C bis etwa 50°C, vorzugsweise bei Raumtemperatur, erfolgen.

Erfindungsgemäss kann zu einer Suspension von Titantetrachlorid und 5-Carbamoyl-4-niederalkyl-oxazol in einem geeigneten Lösungsmittel eine oben genannte Base zugetropft werden. Die Umsetzung mit Titantetrachlorid erfolgt zweckmässigerweise bei einem 5-Carbamoyl-4-niederalkyl-oxazol: Titantetrachlorid-Molverhältnis von etwa 1:1 bis etwa 1:2. Vorzugsweise werden äquimolare Mengen bzw. wird ein geringfügiger Ueberschuss an Titantetrachlorid von 5-10% eingesetzt.

Die Aufarbeitung des 5-Cyano-4-niederalkyloxazols kann durch gängige Verfahren der organischen Chemie, wie z.B. durch Extraktion mit Lösungsmitteln wie Methyltertiärbutylether, Diethylether, Diisopropylether, Tertiäramylmethylether, Ethylacetat, Methylacetat, Isobutylmethylketon, Pentan, Heptan, Dichlormethan und dergleichen, erfolgen.

Mittels des erfindungsgemässen Verfahrens kann eine nahezu quantitative Umsetzung von 5-Carbamoyl-4-niederalkyl-oxazol zu 5-Cyano-4-niederalkyl-oxazol erzielt werden.

Das folgende Beispiel zur Herstellung von 5-Cyano-4-methyl-oxazol zeigt eine besonders vorteilhafte Ausführungsform des erfindungsgemässen Verfahrens auf und soll keinerlei Einschränkung darstellen.

### Beispiel:

Eine Suspension von 24 ml (220 mMol) Titantetrachlorid in 200 ml Acetonitril oder in 200 ml Tetrahydrofuran wird bei Raumtemperatur mit 25.2 g ( 200 mMol) 5-Carbamoyl-4-methyl-oxazol versetzt. Anschliessend werden innerhalb einer Stunde 61.3 ml ( 440 mMol ) Triethylamin zugetropft.

Zur Aufarbeitung wird die Suspension mit 50 ml Wasser hydrolysiert und mit 100 ml Methyltertiärbutylether extrahiert. Die wässrige Phase wird zweimal mit je 50 ml Methyltertiärbutylether extrahiert, und die vereinigten organischen Phasen werden mit 50 ml gesättigter wässriger Natriumchloridlösung gewaschen, über wasserfreiem Natriumsulfat getrocknet, filtriert und am Rotationsverdampfer eingeengt.

Die Ausbeute an 5-Cyano-4-methyl-oxazol beträgt 97% der Theorie.

## Patentansprüche

1. Verfahren zur Herstellung eines 5-Cyano-4-niederalkyl-oxazols durch Dehydratisierung eines 5-Carbamoyl-4-niederalkyl-oxazols, dadurch gekennzeichnet, dass die Dehydratisierung mit Titantetrachlorid in Gegenwart einer Base und gegebenenfalls in Gegenwart eines Lösungsmittels durchführt wird.

2. Verfahren gemäss Anspruch 1, dadurch gekennzeichnet, dass die Base Triethylamin ist.

3. Verfahren gemäss Anspruch 1 oder 2, dadurch gekennzeichnet, dass die Dehydratisierung bei Temperaturen von etwa 0° bis etwa 50°C, vorzugsweise bei Raumtemperatur, erfolgt.

4. Verfahren gemäss einem der Ansprüche 1 bis 3, dadurch gekennzeichnet, dass die Dehydratisierung bei einem 5-Carbamoyl-4-niederalkyl-oxazol : Titantetrachlorid-Molverhältnis von etwa 1:1 bis etwa 1:2 erfolgt.

5. Verfahren gemäss Anspruch 4, dadurch gekennzeichnet, dass 5-Carbamoyl-4-niederalkyl-oxazol und Titantetrachlorid in äquimolarer Menge bzw. ein geringfügiger Ueberschuss an Titantetrachlorid von 5-10% eingesetzt wird.

6. Verfahren gemäss einem der Ansprüche 1 bis 5, dadurch gekennzeichnet, dass die Dehydratisierung in Gegenwart von Tetrahydrofuran oder Acetonitril als Lösungsmittel durchgeführt wird.
